# EUROPEAN PATENT APPLICATION

(11) **EP 3 150 231 A1**
(43) Date of publication of application: **05.04.2017**
(21) Application number: 16156259.0
(22) Date of filing: 18.02.2016
(51) Int. Cl.: A61K 47/69, A61K 31/198, A61P 25/00, B82Y 5/00

(54) **AGENT FOR THE TREATMENT AND PREVENTION OF AUTISM SPECTRUM DISORDERS**

(30) Priority: 02.10.2015 RU 2015141927
(71) Applicant: ZAO "ALMAZ PHARM", 117393 Moscow (RU)
(72) Inventor: LEONIDOV, Nikolay, 117036 Moscow (RU); VORONINA, Tatiana, 119048 Moscow (RU); YAKOVLEV, Rusian, 300001 Tula (RU)
(74) Representative: Jeck, Anton

(57) **Abstract**

The invention relates to medicine, and more particularly to psychopharmacology, and is directed to an agent for treating and preventing autism spectrum disorders; said agent is glycine immobilized on detonation nanodiamond particles 2-10 nm in size, wherein the content of glycine is up to 21±3 wt.%. The described agent improves the outcome of drug therapy and preventive treatment of autism in children and adults and expands the range of the effective and safe psychotropic medications.

## Description

The present invention relates to medicine, more particularly to psychopharmacology, and directed to the known agent (composition) glycine immobilized on detonation nanodiamond particles 2-10 nm in size [1] (hereinafter referred to as "nanolycine") used for the treatment and prevention of various types of autism spectrum disorders regardless of the cause.

Autism spectrum disorders comprise a range of complex mental disorders including social deficits, communication difficulties, and stereotyped behaviors. The patients afflicted with autism characteristically exhibit phobias, agitation, eating disorders, and other nonspecific symptoms [2].

Autism as a condition was first described as recently as 60 years ago, but at the time, this condition was rarely controlled. Today, the number of children afflicted with autism around the world has reached one in 200 (for example, in the UK, one in every 60 children is afflicted), and 75 % of them are mentally delayed [3]. Autism is 3-4 times more common in boys than in girls. This condition affects all socioeconomic classes and has been diagnosed in every country in the world that has conducted corresponding studies [2, 3].

Until now, there haven't been found any pharmaceutical agents or methods for the treatment of autism spectrum disorders [2-4]. However, there are methods that can help individuals with autism to some extent. Moreover, the best results can be achieved only by a simultaneous application of several methods. One of the methods involves treating autism with medications. However, presently, there are no known approved medications for the treatment of autism. In present day psychopharmacology, the treatment of autism with drugs is rather an arbitrary notion; it would be more accurate to talk about "correction" of autism spectrum disorders or treatment of individual autism symptoms. Treating autism with drugs is an important but auxiliary component in the overall treatment plan. Even though the present state of psychopharmacology does not allow for early casual treatment of young autistic children with medications, some characteristics or symptoms of autism disorders, as well as combinations of symptoms can be corrected. For instance, medications can positively affect the motor agitation or self-injurious behavior of an individual with autism, they can neutralize or reduce psychomotor and depressive disorders, affect speech development, etc. In addition, medications are crucial during a crisis intervention [3]. Administration of medications at the utmost early stage is extremely important as a prognostic beneficial factor due to the brain development and positive ontogenesis trends in the cessation of the active disease progression. The medications are selected based on the psychopathological structure of the disorder and the presence or absence of concomitant psychological, neurological, and somatic disorders [3,4].

While treatment of adults is not only focused on liquidation of the manifestations of the disease but on the optimal adjustment to the environment, the treatment of children that benefits their adaptation at every given moment will later contribute to the more adequate psychiatric development [3].

Psychopharmacotherapy of autism spectrum disorders is further complicated, first and foremost, by the fact that the new generation drugs (atypical neuroleptics, antidepressants), as a rule, are not approved for use in children. The first preparation for treating self-injury and anger outbursts is the atypical antipsychotic drug Risperidone that was approved for children with autism in the USA in 2006 [4], and being moderately efficient, is better tolerated than such reference preparation as Haloperidol. Drug choices for treating autism spectrum disorders are therefore quite limited [3, p. 127; 4]. That is also due to severe side effects, including extrapyramidal disorders, caused by most frequently used antipsychotic therapeutic agents. Antidepressants, (clomipramine, amitriptyline, sertraline, and fluoxetine) may also trigger various aggravated positive thought disorders. Autistic anxiety disorders, sleep disorders, obsessive-compulsive behavior, pronounced anxiety with intoxication symptoms are treated with anxiolytics (tranquilizers) and hypnotics, which also cause many toxic and side effects. Anticonvulsants (sodium valproate, carbamazepine, Lamictal, Convulex) are often used if the clinical picture presents pronounced affective disorders. In resent years, the glutamatergic preparation Akatinol Memantine has been used for pathogenically substantiated casual therapy of autistic disorders. These preparations, as a rule, also cause various side effects and affect hematogenesis. Nootropics and the substances exhibiting nootropic behavior are also widely used for the treatment of all types of autistic disorders. Cognitive deficiency is treated with neuroleptics in combination with immune preparations (Kagocel, Tenanten, etc.). Homeopathic agents are also widely used (Cerebrum compositum, Coenzyme compositum, etc.), as a rule, parenterally [3, p. 129-131]. The efficacy of these agents, however, is not very high.

Numerous attempts to find effective preparations and successful methods for the treatment of autistic disorders have not yet yielded any positive results [2-4]. Thus, development and safe application of effective pharmacological agents for the treatment and prevention of autism remains a very important and vital social and medical problem.

Autism spectrum disorders are diagnosed by the presence of certain criteria, the major of which are as follows:
- Stereotyped repetitive behavior, insistence on the maintenance of the same identical conditions;
- Impaired social interactions;
- Impaired learning and, especially, relearning processes;
- Impaired communication skills;
- Limited interests;
- Increased anxiety, etc.

Based on these criteria, in recent years, several special psychopharmacological experimental autism animal models have been developed to reproduce the central nervous system (CNS) disorders in experiments on mice [5-13].

The most commonly known and widely used tests on animals are:
1) Tests evaluating repetitive, insistent (stubborn) motor behavior;
2) Tests evaluating communicative behavior based on olfactory interactions.

The nonessential amino acid glycine (NH₂CH₂COOH) is known to take part in the formation of the most important biologically active compounds: purine nucleotides, heme, creatine, etc. as a central inhibitory neurotransmitter, and furthermore, acts as a sedative, improves the metabolic processes in brain tissue, regulates the formation of fine motor skills of plastic processes and tonic reactions in the somatic musculature [14, 15]. Most of glycine is concentrated in the spinal marrow, wherein the amino acid released from the Renshaw cell endings mediates the postsynaptic release (inhibition) of motor neurons. Glycine, therefore, is widely used in neurological practice to reduce the increased muscle tone.

Glycine is also responsible for the regulation of the NDMA-glutamate receptor activity. It has its own site in most glutamate activating receptors. Reacting with magnesium, glycine acts as an inhibitor; when it is free, it acts as a stimulating agent.

Glycine is used in modern psychopharmacotherapy for alleviating depressive disorders, increased irritability, and alcohol addiction; for relieving withdrawal symptoms, normalizing sleep, enhancing antipsychotic therapy; and also in combination therapy of cerebrovascular disorders [14]. The pharmacological effect of glycine is based on the amplification of metabolic and neurotransmitter functions triggered by the increase of its endogenic synthesis. Intracellular glycine synthesis can only be enhanced via cellular pathways mediated by their interaction with the receptor systems. Interaction of glycine with glycine receptors opens chlorine channels, hyperpolarizes the membrane and spreads out the inhibitory effect. Moreover, glycine can act as an allosteric coagonist of glutamate receptors. During its site-specific binding, it enhances the glutamate's and N-methyl-D-aspartate's (NMDA) ability to open a cation channel [15,16]. Pharmaceutical grade glycine is administered as 0.1 g tablets, sublingually (under the tongue), 3 - 4 times a day [14].

Glycine immobilized on detonation nanodiamond particles 2-10 nm in size [1] (hereinafter referred to as "nanolycine) exhibiting sedative, antidepressant, antipsychotic, or anxiolytic activity at different doses, which greatly exceeds the corresponding types of the specific activity of pharmaceutical grade glycine (active pharmaceutical ingredient), and a method for producing thereof are known in the art [17-20]. The antidepressant effect of nanolycine is at least as great as that of the reference antidepressants amitriptyline and fluoxetine. Moreover, in doses exceeding the recommended therapeutic dose of glycine 20-fold, it did not cause any side or toxic effects.

The use of glycine immobilized on detonation nanodiamond particles (nanolycine) for treating and preventing autism spectrum disorders has not been reported in the scientific or patent literature.

The object of the present invention is to use glycine immobilized on detonation nanodiamond particles 2-10 nm in size (nanolycine) for the treatment and prevention of autism spectrum disorders with no side or toxic effects; to increase the psychopharmacological activity of glycine; and to expand the range of medications used for treating and preventing autism in children and adults.

In accordance with the invention, an agent for the treatment and prevention of autism spectrum disorders is provided, wherein said agent is glycine immobilized on detonation nanodiamond particles 2-10 nm in size, wherein the content of glycine is up to 21±3 wt. %.

In order to evaluate the prospective use of glycine immobilized on detonation nanodiamond particles 2-10 nm in size (nanolycine), wherein the content of glycine is up to 21±3 wt. %, as an agent for the treatment and prevention of autism spectrum disorders, the specific psychopharmacological effect of glycine was compared to that of the pharmaceutical grade glycine and the reference atypical neuroleptic Triftazin (comparator drug).

Specific psychopharmacological effects of nanolycine and comparator drugs were studied in the olfactory habituation/dishabituation test simulating impairment of an adequate response to olfactory stimulation, response to the spatial skill acquisition, and the ability to perform new spatial moves after retraining in a water maze, conducted on 330 male Balb/C mice, age 5-7 weeks, 54 outbred male mice age 2 months, and 18 C57BL/6 male mice, age 2-3 months. The study was conducted according to methods [20,7], respectively.

Statistical data from the experiment was processed with "Statistica v6.0" software using the one-way ANOVA test, nonparametric test for variables (Mann-Whitney U test), and paired Student's t-test for intra-group comparison.

The results of the conducted pharmacological study unexpectedly revealed that:
1) A single administration of nanolycine at a 1-mg/kg dose resulted in faster adaptation of the mice to repeated olfactory stimuli and improved the new social odor recognition. Nanolycine at a 10-mg/kg dose also resulted in faster adaptation of the animals to the olfactory stimuli, but behavior of the animals receiving nanolycine in response to new odors did not differ from the behavior of the control animals. The comparator drug Triftazin (0.5 mg/kg) also improved the new order recognition and accelerated adaptation thereto. The efficacy of nanolycine at both doses was no worse than that of the comparator drug Triftazin. The pharmaceutical grade glycine (active pharmaceutical ingredient) at a 10-mg/kg dose was not very effective in this experiment and was much less effective than 1- and 10-mg/kg doses of nanolycine. Detonation nanodiamonds did not improve the animals' ability to recognize new odors;
2) Nanolycine at a 10-mg/kg dose (administered daily over a 6-day period) significantly improved the learning process of mice in a water maze both prior and post spatial reversal, while at a 1-mg/kg dose, it improved the learning process of mice only on the second day of training. The comparator drugs (administered daily over a 6-day period) Triftazin (0.5 mg/kg) and pharmaceutical grade glycine (10-mg/kg) had no effect on the animals' learning process, same as the detonation nanodiamonds.

The invention is illustrated by the following examples:

### Example 1. Comparison study of the effect of nanolycine, glycine, Triftazin, and nanodiamond on the characteristics of autism in the olfactory habituation/dishabituation test.

Autism impairs the ability to adequately react to social and nonsocial olfactory stimuli. Experimental models of said disorders take into consideration the established fact that all interactions between mice, first and foremost, occur via their olfactory signals. Thus, the olfactory habituation/dishabituation in the experiment is examined by repeatedly exposing the mice to nonsocial and social odors in the habituation/dishabituation test [5, 12, 21].

The experiment was conducted on male Balb/C mice, 5-7-weeks old, weighing 13-15 g. C57BI/6 mice of the same gender as the tested animals were used as social stimuli. Outbred male mice, 2-3 month old, weighing 24-30 g were used as an additional control.

The animals were received from the RAMS nursery "Stolbovaya" (Moscow Oblast). The animals were kept in a vivarium in accordance with torder #708n of the Ministry of Health and Social Development of the Russian Federation, August 23, 2010 "On approval of Good Laboratory Practice". The animals were allowed free access to food and water and were fed a full ration of extruded pelletized feed (GOST feed P50258-92) and drinking water. The temperature was maintained at 20-22°C with the light-dark cycle of 12 hours of light and 12 hours of darkness. The animals were kept in polypropylene cages with zinc/chromium steel grates and dust-free litter of wood shavings, 10 mice per cage (T/3C). The mice were kept in accordance with normative document #1045-73, 04.06.1973 "Sanitary Regulations for Arrangement, Equipment, and Maintenance of Vivariums" approved by the Chief Public Health Official.

The following substances were used in Example 1:
- glycine immobilized on detonation nanodiamond particles 2-10 nm in size, wherein the content of glycine was up to 21±3 wt. % (nanolycine) at 1- and 10-mg/kg doses;
- detonation nanodiamonds in concentrations corresponding to the 10-mg/kg doses of glycine;
- pharmaceutical grade glycine (comparator drug) at a 10-mg/kg dose;
- Triftazin (comparator drug) in a 0.5 mg/kg dose.

The substances were administered to the mice once, intraperitoneally, at 0.1 ml per 10 g body weight, 40 min. prior to the experiment.

Control Balb/C animals and outbred mice were intraperitoneally administered 0.1 ml of physiological solution per 10 g body weight once, 40 min. prior to the experiment.

The olfactory habituation/dishabituation test was used to determine the animals' ability to respond to social and nonsocial olfactory stimuli. The test was conducted according to method [21] proposed in 2010.

*Experimental procedure.* Prior to testing, the mice were placed separately into a cage with clean wood shavings for 30 min. A 15-cm long cotton swab was moistened with water and extended through the cage cover to the height of 5 cm above the wood shavings on the bottom of the cage. Each odor was introduced three times:
3 introductions - water;
3 introductions - nonsocial stimulus: diluted flower smell (lemon extract diluted in a 1:100 ratio (water);
3 introductions - social stimulus (odor from an "unfamiliar" murine cage with soiled wood shavings).

For the social stimulus, a cotton swab that had been moistened with water was used to swab the bottom of "another" soiled murine cage, zigzagging through the cage to cover every corner and the center of the cage. The "other" cage should have housed at least 3 C57BI/6 mice of the same gender as the tested mice. The wood shavings could not be replaced for at least 3 days.

The observation was conducted for a period of 2 min. The following parameters were recorded:
- number of head (nose) lifts and turns toward the swab with an odor located no farther than 2 cm away;
- number of approaches to the swab;
- number of sniffs of the swab;
- number of climbs onto the swab;
- number of chewings of the swab.

After 2 min., the cotton swab was replaced with another, which was also introduced for 2 min.

### Experimental results.

*Behavior of outbred and Balb*/*C mice exposed to olfactory stimuli based on the responses to the olfactory stimuli.*

The characteristic feature of the intact outbred mice behavior during the entire experiment was their recognition of a new odor, which was revealed in their heightened reaction to the swab with the odor at the first exposure to each olfactory stimulus and habituation to the odor at the repeated exposure.

The first testing of the "lemon" odor in outbred mice showed a 23.4% increase in the response of mice to the olfactory stimulus as compared to their previous exposure to the neutral "water" smell (third exposure), which had already become habitual. At the first exposure to the olfactory social stimulus "C57BI/6 mice odor" as compared to the third exposure to the "lemon" smell, which at that moment became habitual, the response of the mice to the swab with the odor increased 5.5 fold (Table 1).

The pattern of the murine response to a repeated exposure to an odor is shown in Table 1. The Table demonstrates that the number of outbred mice that responded to the olfactory stimulus "water" at the second exposure was 24.2 % lower than the number of mice at the first exposure. When tested with "lemon" and "C57BI/6 mice" odors, the animals' response was reduced more significantly, at 74.1 % and 51.6 %, respectively.

**Table 1. Response of Balb/C mice to the olfactory stimuli as compared to the outbred white mice (responses include head turns toward the swab with an odor located no farther than 2 cm away; sniffing, climbing, chewing, or approaching the swab).**

| Olfactory stimuli | # of exposures to the odor | Response to olfactory stimulus, units | |
|---|---|---|---|
| | | Control, Outbred mice n = 15 | Control, Balb/C mice, n = 15 |
| Water | 1 | 6.2±0.7 | 6.3±1.0 |
| | 2 | 4.7±0.8 | 4.0±0.9 |
| | 3 | 4.7±0.8 | 6.0±0.6 |
| Lemon | 1 | 5.8±0.9 | 2.8±0.5 |
| | 2 | 1.5±0.3* | 1.6±0.4 |
| | 3 | 1.7±0.5 | 1.0±0.3 |
| C57BI/6 mice odor | 1 | 9.3±0.9 | 7.0±1.0 |
| | 2 | 4.5±0.4* | 5.2±0.8 |
| | 3 | 2.5±0.4# | 2.0±0.5# |

| | | | |
|---|---|---|---|
| * - p<0.05 statistically significant difference as compared to the values at the first measurement of the same stimulus; # - p<0.05 statistically significant difference as compared to the values at the second measurement of the same stimulus. | | | |

At the second exposure to the "lemon" and "C57BI/6 mice" odors, Balb/C mice showed less pronounced adaptation to the olfactory stimuli than the outbred mice group. Thus, the second exposure to the "lemon" and "C57BI/6 mice" odors of Balb/C mice showed a reduced response, by 42.9 % and 25.7 % respectively, which was 1.7 and 2.0 respectively lower than the outbred mice group (Table 1).

The ability of Balb/C mice to recognize the olfactory stimuli "water" and "C57BI/6 mice odor" did not differ from that of the outbred mice, which was confirmed by the absence of difference between the responses to new odors in both control groups. The response to the "lemon" odor, however, in Balb/C mice was 2.1 times lower than that of the outbred animals (Table 1).

### The effect of nanolycine on the behavior of Balb/C mice at the exposure to olfactory stimuli based on the response to the olfactory stimuli.

Balb/C mice that were administered 1- and 10-mg/kg doses of nanolycine, developed habituation to the "water" and "C57BI/6 mice odor" olfactory stimuli after repeated exposures faster than the Balb/C controls (Table 2). Indeed, in the "nanolycine 1-mg/kg" group, the reduced murine response to the "water" and "C57BI/6 mice odor" olfactory stimuli after repeated exposures was more statistically significant than after the first exposure: 32.5 % 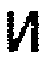 61.2 %, respectively. In the "nanolycine 10-mg/kg" group the reduced murine response to the "water" and "C57BI/6 mice" odors was 43.4 % and 65.4 % (p<0,05). A reduced murine response after the third exposure to the "water" and "C57BI/6 mice odor" olfactory stimuli in the "nanolycine, 1-mg/kg" group followed the trend. In addition, the response of the animals receiving nanolycine at a 10-mg/kg dose to the third "lemon" odor exposure was statistically significant: 68 % lower than their response to the second exposure.

Furthermore, the ability of the animals receiving nanolycine at a 1-mg/kg dose, to recognize the "C57BI/6 mice odor" stimulus at the first exposure was 2.1 times higher than that of Balb/C mice (p<0,05).

Nanodiamond did not alter the murine ability to recognize olfactory stimuli as compared to the control. However, the animals receiving nanodiamond developed habituation to the "water" and "C57BI/6 mice odor" stimuli faster as compared to the Balb/C control group animals (Table 2).

**Table 2. Effects of nanolycine on the Balb/C mice behavior at the exposure to olfactory stimuli as compared to glycine, Triftazin, and nanodiamond in the olfactory habituation/dishabituation test (responses include murine head turns toward the swab with an odor located no farther than 2 cm away, sniffing, climbing, chewing, or approaching the swab).**

| Olfactory stimuli/# of exposures to the odor | | Response to the olfactory stimulus of Balb/C mice, units. | | | | | |
|---|---|---|---|---|---|---|---|
| | | Control Balb/C mice, n=15 | Nanolyci ne 1-mg/kg, n=15 | Nanolyci ne 10-mg/kg, n=15 | Nanodiam ond n=15 | Glycine 10-mg/kg, n=15 | Triftazin 0.5 mg/kg, n=15 |
| Water | 1 | 6.3±1.0 | 7.7±0.8 | 8.3±0.8 | 7.0±0.6 | 10.2±0.9 | 8.0±0.6 |
| | 2 | 4.0±0.9 | 5.2±0.9* | 4.7±0.8* | 5.5±0.6 | 6.2±0.9* | 4.6±0.4* |
| | 3 | 6.0±0.6 | 3.3±0.4 | 3.8±0.6 | 3.5±0.7# | 4.0±1.0 | 1.6±0.4# |
| Lemon | 1 | 2.8±0.5 | 2.0±0.4 | 2.5±0.6 | 2.7±0.6 | 5.8±0.5 | 3.8±1.0 |
| | 2 | 1.6±0.4 | 1.3±0.4 | 2.5±0.7 | 2.5±0.6 | 2.6±0.6 | 0.4±0.2* |
| | 3 | 1.0±0.3* | 1.3±0.4 | 0.8±0.2# | 1.7±0.5 | 1.0±0.3# | 0.0±0.0 |
| C57BI/ 6 mice odor | 1 | 7.0±1.0 | 14.7±1.6 $ | 7.8±0.8 | 8.3±0.7 | 10.8±0.9 | 9.8±1.2 |
| | 2 | 5.2±0.8 | 5.7±1.2* | 2.7±0.7* | 2.5±0.5* | 5.0±0.7* | 5.2±0.7* |
| | 3 | 2.0±0.5# | 3.3±0.5 | 1.5±0.3 | 2.5±0.6 | 2.2±0.5# | 2.6±0.8# |

| | | | | | | | |
|---|---|---|---|---|---|---|---|
| * - p<0.05 statistically significant difference as compared to the values at the first measurement of the same stimulus; # - p<0.05 statistically significant difference as compared to the values at the second measurement of the same stimulus. $ - p < 0.05 statistically significant difference as compared to the values in the "glycine as an active pharmaceutical ingredient at 10-mg/kg" group. | | | | | | | |

When comparator drugs glycine at a 10-mg/kg dose and Tritazin, at a 0.5 mg/kg dose, were administered, habituation to olfactory stimuli also occurred faster as compared to the control Balb/C animals.

A reduced response to the second exposure of the animals receiving glycine at a 10-mg/kg dose to the neutral olfactory stimulus "water" was statistically significant (39.2 %) in comparison to the first exposure. Glycine caused significantly faster habituation to each consecutive exposure to the "C57BI/6 mice odor" olfactory stimulus. A response to the third exposure to the "lemon" odor in this group was also significantly reduced in comparison to the second exposure. All in all, this group of animals showed more statistically significant (p<0,01) pronounced response to the recognition of all olfactory stimuli as compared to the control Balb/C animal group.

The second comparator drug Triftazin caused significantly faster habituation to each consecutive exposure to the "water" and "C57BI/6 mice odor" olfactory stimuli. Furthermore, a response of the mice receiving Triftazin to the third exposure to the "lemon" odor was significantly (9.5 times) lower than their response to the second exposure. Although recognition of all olfactory stimuli by all the animals in this group at the first exposure was more pronounced in comparison to the control, it was not statistically significant (Table 2).

Thus, nanolycine at 1- and 10-mg/kg doses improves the recognition of new social odors and adaptation to olfactory stimuli in the olfactory habituation/dishabituation test. nanolycine at both doses is as effective as the comparator drug Triftazin (0.5 mg/kg) and more effective than glycine (10-mg/kg). Detonation nanodiamonds do not improve the murine ability to recognize new odors in comparison to the control.

### Example 2. Comparison study of the effect of nanolycine vs. glycine, Triftazin, and nanodiamonds on the autism manifestations in learning and relearning tests following spatial "reversal" in the Morris water maze.

Obsessive repetitive stereotyped behavior, limited interests and activities are typical characteristics of autism [22, 5]. Repetitive stereotyped behavior in mice includes rotating motion, turning back, repeated sniffing of the same places or objects, excessive grooming, and excessive purposeless motor activity [23, 8].

Repetitive obsessive behavior of Balb/C mice is revealed in the animals' inability to make a decision and choose the correct spatial move when the platform is relocated during retraining in a water maze [8].

The experiment was conducted on male Balb/C mice, 5-7-weeks old, weighing 13-15 g. The source of mice and holding conditions were identical to those in Example 1.

In Example 2, the tested substances were used the same way as in Example 1.

The substances were administered to the mice at 0.1 ml per 10 kg of body weight over 6 days. The control Balb/C animals and outbred mice were intraperitoneally administered physiological solution at 0.1 ml per 10 kg of body weight 40 min. prior to the experiment.

A method developed in 2007 and described in [7] was used to evaluate the effect of nanolycine on the symptoms of autism spectrum disorders. The effect of the tested compounds on the spatial learning process and the ability to make a new spatial move during retraining in a water maze were examined.

The test was conducted in the Morris water maze. The mice were first taught the skill of locating a platform in a water maze and replicating this spatial skill. The animals were later confronted with a new location of the platform and thus, spatial "reversal" was created. During relearning (relocation of the platform in the water maze) the animals had to make a new decision and make a correct spatial move.

The Morris maze is a large circular pool, 122 cm in diameter, 25 cm deep, filled with water at 25-28°C. A round platform 12 cm in diameter was placed into the pool. The center of the platform was positioned 30 cm away from the edge of the pool.

### Experimental procedure.

1st stage. Getting familiar with experimental conditions. A platform is located 0.5 cm above the water. A mouse is placed on the platform for 20 sec. The mouse is then placed in the water at the opposite end of the pool and allowed 60 sec. to find the platform, climb it, and stay there for 20 sec. The process is repeated by placing the mouse in the water in the location of the pool different from the location of the first attempt. Each animal is allowed 4 attempts to find the platform.

If the animal is unable to find the platform all by itself in 60 sec., the researcher helps it to find its way to the platform and climb it.

2nd stage. Teaching spatial skills to the animals. During the next 2 days, the platform is placed 0.5 cm below the water level. The animals are allowed 4 attempts per day to find the platform in 60 sec. The time gap between the attempts is 20 sec., wherein they stay on the platform. Every day, before their first attempt, the animals are placed on the platform for 20 sec. The time between the moment the animal is placed in the water and the moment the animal climbs on the platform is recorded, as well as the number of effective attempts to find the platform. The animals are placed in the water in three different locations in the part of the pool opposite the platform.

3rd stage. Replicating the spatial skill. 24 hrs. post the second day of training, the replication of the spatial skill is evaluated: the platform is removed and the animals are placed in the pool for 60 sec. once; the time length of the animal's stay in the quadrant wherein the platform had been located during the learning stage is recorded. Said time is an indicator of learning efficiency and replication of the spatial skill.

4th stage. Reversal in the spatial skill - relearning and replication. The day after replicating the spatial skill, the platform is moved to the area of the pool diagonally opposite its previous location. The platform has to be immersed in the water at the same 0.5 cm depth below the water level as in the previous days of training. The training (2 days) and replication procedures are repeated according to the scheme described earlier.

### Results of the experiments.

### Effect of nanolycine on learning and replicating the spatial skill in Balb/C mice in the Morris water maze.

In the first day of training in the Morris water maze, Balb/C mice were reported to show a statistically significant increase in the time of the platform search (by 24.0 %) and a decreased number of the effective attempts (3.6 times) as compared to the outbred mice (Table 3).

Balb/C mice receiving nanolycine at 1- and 10-mg/kg doses, nanodiamond, glycine, and Triftazin, didn't show any statistically significant difference from the linear mice. The trend was (p < 0.1, Student's t-test) toward reduction in the time searching for the platform in the group of mice receiving nanolycine at a 1-mg/ml dose as compared to the control Balb/C mice (Table 3).

**Table 3. Effect of nanolycine, glycine as an active pharmaceutical ingredient, Triftazin, and nanodiamond on learning and replicating the spatial skill in the Morris water maze.**

| Groups | Training | | | | Replication |
|---|---|---|---|---|---|
| | Time of searching for the platform, sec. | | Number of effective attempts, units. | | Time in the platform quadrant, sec. |
| | 1 day | 2 day | 1 day | 2 day | |
| Outbred mice, n=15 | 27.7±5.9# | 23.6±4.9# | 3.0±0.5# | 3.4±0.3# | 23.0±2.6# |
| Control Balb/C mice n=15 | 48.8±5.4 | 47.3±5.4 | 0.9±0.3 | 1.5±0.4 | 9.1±1.4 |
| Nanolycine 1-mg/kg, n=15 | 42.0±6.5 | 43.2±4.4 | 2.2±0.6# | 2.7±0.7 | 30.5±4.8# |
| Nanolycine 10-mg/kg, n=15 | 53.5±2.5 | 42.3±5.7* | 1.3±0.3 | 2.3±0.5* | 12.5±3.4 |
| Nanodiamond n=15 | 55.6±1.7 | 52.8±2.6 | 0.5±0.2 | 1.2±0.3 | 14.9±1.8 |
| Glycine, 10-mg/kg, n=15 | 54.5±1.8 | 51.3±3.2 | 0.8±0.3 | 1.7±0.6 | 15.0±5.2 |
| Triftazin, 0.5 mg/kg, n=15 | 55.6±2.3 | 54.1±2.7 | 0.5±0.2 | 0.8±0.3 | 10.8±2.8 |

| | | | | | |
|---|---|---|---|---|---|
| #-p<0.05 as compared to the control Balb/C mice; *- p<0.05 as compared to the parameters recorded on the first training day. | | | | | |

On the second day of training, the linear mice also showed behavior that was significantly different from the behavior of the outbred mice in the time of searching for the platform (72.2 % increase) and in the number of effective attempts (6.4 times reduction). The first and second day results within the Balb/C group didn't differ from one another, while the outbred animals found the platform on the second say of training significantly faster (28.6 %) and made a larger number of effective attempts (1.8 times) as compared to the first training day (Table 3).

The animals receiving nanolycine at a 10-mg/kg dose spent significantly less time (18.5 %) on the search for the platform and made significantly larger number of effective attempts (3.6 times) as compared to the control group. nanolycine at a 10-mg/kg dose had a positive effect on the ability of the animals to learn, which was confirmed by a statistically significant decrease in the time spent on the search of the platform (15.6 %) and an increase in the number of effective attempts (80 %) on the second day as compared to the first day of training.

The 1-mg/kg of nanolycine dose significantly (4.4 times) increased the number of effective attempts to find the platform in comparison to the control group of linear mice. The reduction in the time that took to find the platform for the mice receiving nanolycine was at the trend level (p < 0.1, Student's t-test) and amounted to 14 %.

The comparator drug Triftazin did not make any impact on the training of the animals in the water maze in comparison to the control group. However, within the same group, on the second day of training, Triftazin caused a statistically significant reduction in the time that took to find the platform (12.3 %) and an increase in the number of effective attempts (by 62.5 %) in comparison to the first day (Table 3).

When replicating the spatial skill, the time the linear mice spent in the quadrant, in which the platform was located during training, was significantly (2.3 times) shorter as compared to the outbred animals (Table 3). The time spent in said quadrant for the animals receiving the substances under study did not show a statistically significant difference in comparison to the linear mice group.

### Effect of nanolycine on the behavior of Balb/C mice after spatial reversal (relearning) in the Morris water maze.

The time that took mice to find the platform and the number of effective attempts immediately after "reversal" (relearning process) in the control Balb/C was found to be significantly different from the outbred animal group both in the first and second days of their retraining (Table 4).

The Balb/C groups receiving nanolycine at a 1-mg/kg dose, nanodiamond, glycine, and Triftazin, did not show any statistically significant difference from the control group in both recorded parameters on the first and second day of retraining (Table 4).

The animals receiving nanolycine at a 1-mg/kg dose showed a statistically significant improvement in their retraining. Indeed, on the first day of retraining, the animals made 2.4 times the number of effective attempts to find the platform as compared to the control animals; and on the second day, the number of effective attempts to find the platform was 80 % higher than that of the control group (Table 4).

On the second day of retraining, the mice receiving nanolycine at a 10-mg/kg dose spend significantly less time on the search for the platform and made significantly more effective attempts in comparison to the first day of retraining, which demonstrated the positive effect of the preparation during retraining ("reversal") (Table 4) When replicating the skill acquired after spatial "reversal", the control animals were found to spend significantly less time than the outbred mice in the quadrant, in which the platform was located during retraining (Table 4). nanolycine at a 1-mg/kg dose significantly (3.4 times) increased the time the animals spent in the "correct" quadrant. A statistically significant increase of this parameter was also observed in the group of animals receiving nanodiamond, and reached 90.1 %. The comparator drugs Triftazin and glycine, similar to nanolycine at a 10-mg/kg dose, did not make any impact on the replication of the newly acquired skill.

**Table 4. Effect of nanolycine, glycine, Triftazin, and nanodiamond on learning and replicating the spatial skill after spatial reversal in the Morris water maze.**

| Groups | Training | | | | Replication |
|---|---|---|---|---|---|
| | Time of searching for the platform, sec. | | Number of effective attempts, units. | | Time in the platform quadrant, sec. |
| | 1 day | 2 day | 1 day | 2 day | |
| Outbred mice, n=15 | 45.8±3.4# | 32.7±4.5#* | 1.8±0.4# | 3.2±0.3#* | 22.3±2.6# |
| Control Balb/C mice n=15 | 56.8±1.8 | 56.3±1.8 | 0.5±0.3 | 0.5±0.2 | 9.5±2.6 |
| Nanolycine 1-mg/kg, n=15 | 49.4±3.7 | 48.4±3.9 | 1.2±0.4 | 2.2±0.6# | 11.2±4.5 |
| Nanolycine 10-mg/kg, n=15 | 54.4±3.1 | 45.9±4.5#* | 1.0±0.5 | 1.8±0.5# | 14.1±4.6 |
| Nanodiamond n=15 | 55.1±2.3 | 53.6±2.6 | 0.5±0.3 | 0.7±0.2 | 11.2±3.1 |
| Glycine, 10-mg/kg, n=15 | 51.9±2.6 | 56.5±1.5 | 1.0±0.3 | 1.0±0.4 | 8.2±2.9 |
| Triftazin, 0.5 mg/kg, n=15 | 55.9±1.3 | 49.0±4.4* | 0.8±0.4 | 1.3±0.5 | 15.8±2.8 |

| | | | | | |
|---|---|---|---|---|---|
| #-p<0.05 as compared to the control Balb/C mice; *- p<0.05 as compared to the parameters recorded on the first training day during retraining. | | | | | |

Thus, nanolycine at a 10-mg/kg dose significantly improves the learning process of Balb/C mice in the Morris water maze both before and after spatial reversal. nanolycine at a 1-mg/kg dose improved the learning process of Balb/C mice on the second day only. The comparator drugs Triftazin and glycine did not make any impact on the animals' learning process. None of the investigated preparations made any impact on the replication of the spatial skill. However, when replicating the spatial skill after "reversal" of spatial memory, nanolycine at a 1-mg/kg dose significantly increased the time Balb/C mice spent in the platform quadrant.

The experimental results obtained in Examples 1 and 2 demonstrated that a single administration of the agent of the present invention at 1- and 10-mg/kg doses had a pronounced effect on the social odor recognition and accelerated adaptation to olfactory stimuli in the olfactory habituation/dishabituation test. At both doses, the claimed agent was as effective as the comparator drug Triftazin (0.5 mg/kg) and more effective than glycine (10-mg/kg). Detonation nanodiamond did not improve recognition of new odors. The claimed agent at a 10-mg/kg dose (daily administration over a 6-day period) significantly improved the learning process of Balb/C mice in the Morris water maze both prior and post spatial reversal. The comparator drugs Triftazin (0.5 mg/kg) and glycine (10-mg/kg) and nanodiamonds (administered daily over a 6-day period) did not make any impact on the learning process of the animals. Following reversal of spatial memory, the claimed agent at a 1-mg/kg dose (administered daily over a 6-day period) improved replication of the spatial skill and significantly increased the time the animals spend in the platform quadrant. The comparator drugs Triftazin (0.5 mg/kg) and glycine (10-mg/kg) as well as nanodiamonds (administered daily over a 6-day period) did not make any impact on the animals' learning process. After reversal of spatial memory, the claimed agent at a 1-mg/kg dose (administered daily over a 6-day period) improved replication of the spatial skill, significantly increased the time the animals spend in the platform quadrant. The comparator drugs Triftazin (0.5 mg/kg) and glycine (10-mg/kg) as well as nanodiamonds (administered daily over a 6-day period) did not make any impact on replication of the spatial skill after reversal of spatial memory.

### Literature

1. Yakovlev R.Yu. Nanodiamond-glycine conjugate and method for the preparation thereof. Pat. RF 2560700, 2015. ( 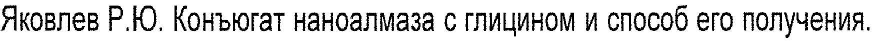 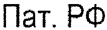 2560700, 2015.)
2. Rational pharmacotherapy in psychiatric practice: Manual for practicing physicians. Edited by Aleksandrovsky Yu.A., Neznanov N.G., M. Littera, 2014 P .714-732 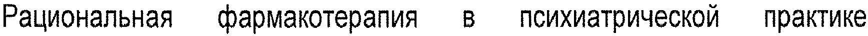 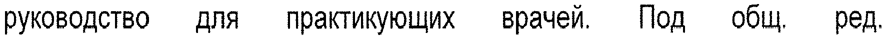 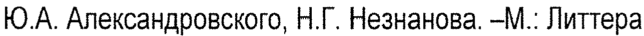 , 2014. C. 714-732.
3. Autism spectrum disorders in children. Scientific and practical manual. Edited by Simashkova N.V. M. Avtorskaya akademiya, 2013, 264 p. 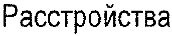 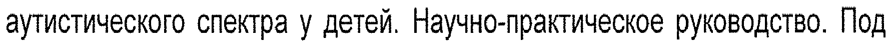 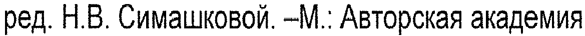 , 2013. 264 c.
4. Manual of clinical psychopharmacology/ Schatzberg A.F., Cole J.O., Debattista C., translated from the English, edited by Smulevich A.B., Ivanov S.V., 2nd edition., M. Medpress-inform, 2014, p. 175-220 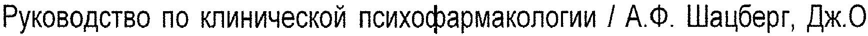 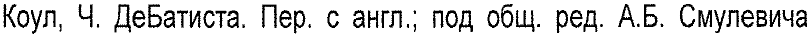 , C.B. 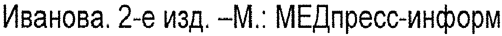 , 2014. C. 175-220.
5. Roullet F.I., Crawley J.N. Mouse Models of Autism: Testing Hypotheses About Molecular Mechanisms / J.J. Hagan (ed.) Molecular and Functional Models in Neuropsychiatry. Curr. Topics in Behav. Neurosci. 2010. V.7. P. 187-212.
6. Moy S.S., Nadler J.J., Perez A. et al. Sociability and preference for social novelty in five inbred strains: an approach to assess autistic-like behavior in mice // Genes Brain Behav. 2004; 3(5):287-302.
7. Moy S.S., Nadler J.J., Young N.B. et al. Mouse behavioral tasks relevant to autism: phenotypes of 10 inbred strains // Behav Brain Res. 2007. V. 176 Nº 1. P. 4-20;
8. Moy S.S., Nadler J.J., Poe M.D. et al. Development of a mouse test for repetitive, restricted behaviors: relevance to autism // Behav. Brain Res. 2008. V. 188. Nº 1. P. 178-194.
9. Nadler J.J., Moy S.S., Dold G. et al. Automated apparatus for quantitation of social approach behaviors in mice // Genes Brain Behav. 2004. V. 3. P. 303-314.
10. Kwon C.H., Luikart B.W., Powell C.M. et al. Pten regulates neuronal arborization and social interaction in mice // Neuron. 2006 V. 50. P. 377-388.
11. Crawley J.N., Chen T., Puri A. et al. Social approach behaviors in oxytocin knockout mice: comparison of two independent lines tested in different laboratory environments // Neuropeptides. 2007 V. 41. Nº 3. P. 145-163.
12. Chadman K.K., Gong S., Scattoni M.L.S. et al.Minimal aberrant behavioral phenotypes of neuroligin-3 R451C knockin mice // Autism Res. 2008. V. 1. P. 147-158.
13. Moon J., Beaudin AE., Verosky S. et al. Attentional dysfunction, impulsivity, and resistance to change in a mouse model of fragile X syndrome // Behav. Neurosci. 2006 V. 120. P. 1367-1379.
14. Mashkovsky M.D., Medications, 16th edition, revised, corrected, enlarges. M. Novaya Volna, publisher: Umerenkov, 2012, p. 661 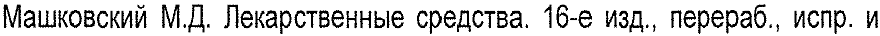 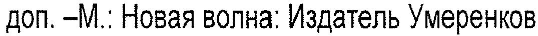 , 2012. C. 661.
15. Komisarova I.A., Nartsissov Ya.R., Molecular mechanisms of action of the pharmaceutical preparation "glycine". Terra medica. 2001. #1. pp. 23-25. 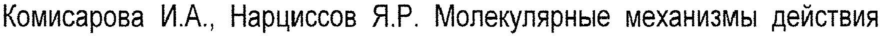 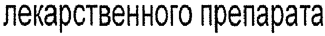 « 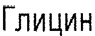 » // Terra medica. 2001. Nº1. C. 23-25.
16. Bespalov A.Yu., Evartau E.E. Neuropsychopharmacology of NMDA-receptor antagonists. St. Petersburg, Nevsky Dialect, 2000, 297 p. 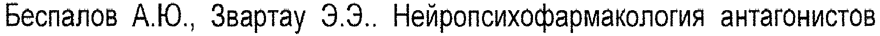 NMDA- 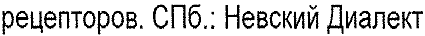 , 2000. 297 c.
17. Leonidov N.B., Yakovlev R.Yu., Lisichkin G.V., Sedative and method for the preparation thereof. Pat. RF. 2506075, 2013. 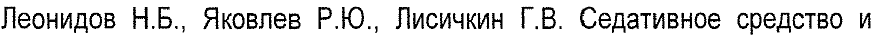 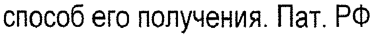 2506075, 2013.
18. Leonidov N.B., Yakovlev R.Yu., Solomatin A.S., Lisichkin G.V., Antidepressant and method for the preparation thereof. Pat. RF. 2519759, 2013. 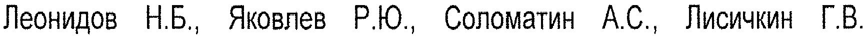 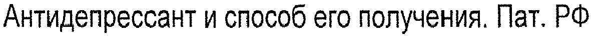 2519759, 2013.
19. Leonidov N.B., Yakovlev R.Yu., Lisichkin G.V., Antipsychotic agent and method for the preparation thereof. Pat. RF. 2519761, 2013. 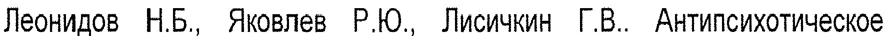 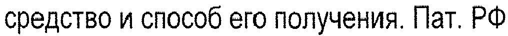 2519761, 2013.
20. Leonidov N.B., Yakovlev R.Yu., Kulakova I.I., Lisichkin G.V., Anxiolytic and method for the preparation thereof. Pat. RF. 2519755, 2013 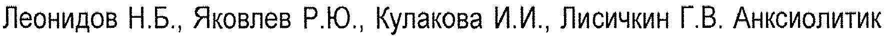 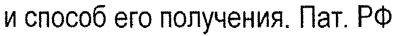 2519755, 2013.
21. Ryan B.C., Young N.B., Crawley J.N., et al. Social deficits, stereotypy and early emergence of repetitive behavior in the C58/J inbred mouse strain // Behav. Brain Res. 2010 V. 208. Nº 1. P. 178-188.
22. South M., Ozonoff S., McMahon W.M. Repetitive behavior profiles in Asperger syndrome and high functioning autism // J. Autism Dev Disord. 2005 V. 35. P. 145-158.
23.Crawley J.N. Mouse behavioral assays relevant to the symptoms of autism // Brain Pathol. 2007 V. 17. P. 448-459.

## Claims

1. A pharmaceutical composition comprising a conjugate of glycine immobilized on detonation nanodiamond particles, 2-10 nm in size, wherein the content of glycine is up to 21±3 wt.%, for the treatment and prevention of autism spectrum disorders.

2. The pharmaceutical composition of claim 1, wherein the autism spectrum disorder is repetitive, persistent (stubborn) stereotyped behavior.

3. The pharmaceutical composition of claim 1, wherein the autism spectrum disorders are impaired learning and relearning processes.

4. The pharmaceutical composition of claim 1, wherein the autism spectrum disorders are impaired communication skills and impaired social interaction.
